(19) Europäisches Patentamt
European Patent Office
Office européen des brevets

(11) Veröffentlichungsnummer: **0 327 924 A1**

# (12) EUROPÄISCHE PATENTANMELDUNG

(21) Anmeldenummer: **89101610.7**

(22) Anmeldetag: **31.01.89**

(51) Int. Cl.⁴: **C07C 155/02 , C07C 155/03 , C07C 155/08 , C07C 155/09 , A01N 47/24**

(30) Priorität: **11.02.88 DE 3804195**

(43) Veröffentlichungstag der Anmeldung: **16.08.89 Patentblatt 89/33**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Müller, Nikolaus, Dr.**
**Knipprather Strasse 98**
**D-4019 Monheim(DE)**
Erfinder: **Andrews, Peter, Dr.**
**Gellertweg 2**
**D-5600 Wuppertal 1(DE)**
Erfinder: **Harder, Achim, Dr. Dr.**
**Auf dem Bruch 49**
**D-5090 Leverkusen 3(DE)**

## (54) Verwendung von schwefelhaltigen N-Organooxycarbamidsäureestern zur Bekämpfung von Endoparasiten, neue N-Organooxycarbamidsäureester und Verfahren zu ihrer Herstellung.

(57) Die vorliegende Erfindung betrifft die Verwendung von schwefelhaltigen N-Organooxycarbamidsäureestern der Formel I

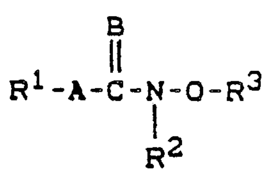

I

in welcher
A und B für O oder S stehen, mit der Maßgabe, daß mindestens einer der Reste A oder B für S stehen muß,
$R^1$ für Alkyl, Cycloalkyl, Alkenyl, Alkinyl, Aryl steht, die gegebenenfalls substituiert sein können,
$R^2$ für Alkyl, Alkenyl, Alkinyl steht, die gegebenenfalls substituiert sein können,
$R^3$ für Alkyl, Alkenyl, Alkinyl steht, die gegebenenfalls substituiert sein können,
zur Bekämpfung von Endoparasiten in der Medizin und Tiermedizin, sowie neue schwfelhaltige N-Organooxycarbamidsäureester und Verfahren zu ihrer Herstellung.

EP 0 327 924 A1

# Verwendung von schwefelhaltigen N-Organooxycarbamidsäureestern zur Bekämpfung von Endoparasiten, neue N-Organooxycarbamidsäureester und Verfahren zu ihrer Herstellung

Die vorliegende Erfindung betrifft die Verwendung von schwefelhaltigen N-Organooxycarbamidsäureestern zur Bekämpfung von Endoparasiten, neuen N-Organooxycarbamidsäureester und Verfahren zu ihrer Herstellung.

N-Organocarbamidsäureester sind bereits bekannt. Es ist jedoch nichts über ihre Verwendung gegen Endoparasiten bekannt.

Alkyl- und Phenylcarbonyl-substituierte Hydroxylamine und ihre anthelmintische Wirkung sind bereits bekannt. Ihre Wirkung ist jedoch nicht in allen Fällen befriedigend.

1. Es wurde nun gefunden, daß schwefelhaltige N-Organooxycarbamidsäureester der Formel I,

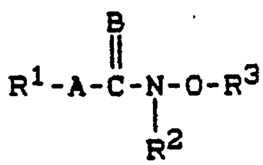

I

in welcher

A und B für O oder S stehen, mit der Maßgabe, daß mindestens einer der Reste A oder B für S stehen muß,

$R^1$ für Alkyl, Cycloalkyl, Alkenyl, Alkinyl, Aryl steht, die gegebenenfalls substituiert sein können,

$R^2$ für Alkyl, Alkenyl, Alkinyl steht, die gegebenenfalls substituiert sein können,

$R^3$ für Alkyl, Alkenyl, Alkinyl steht, die gegebenenfalls substituiert sein können,

sich zur Bekämpfung von Endoparasiten in der Medizin und Tiermedizin eignen.

Die Verbindungen der Formel I sind teilweise bekannt und lassen sich analog zu bekannten Verfahren herstellen.

2. Es wurden neue schwefelhaltige N-Organooxycarbamidsäureester der Formel I gefunden,

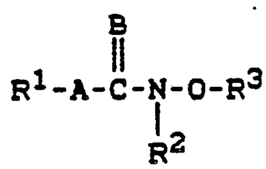

I

in welcher

A und B für O oder S stehen, mit der Maßgabe, daß mindestens einer der Reste A oder B für S stehen muß.

$R^1$ für Alkyl, Cycloalkyl, Alkenyl, Alkinyl, Aryl steht, die gegebenenfalls substituiert sind,

$R^2$ für Alkyl, Alkenyl, Alkinyl steht, die gegebenenfalls substituiert sind,

$R^3$ für Alkyl, Alkenyl, Alkinyl steht, die gegebenenfalls substituiert sind mit der Maßgabe, daß mindestens einer der Reste $R^2$ oder $R^3$ für Propargyl steht.

3. Schwefelhaltige N-Organooxycarbamidsäureester der Formel I,

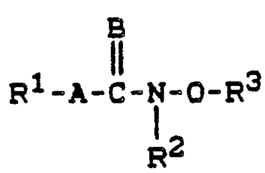

I

in welcher

A und B für O oder S stehen, mit der Maßgabe, daß mindestens einer der Reste A oder B für S stehen muß.

$R^1$ für Alkyl, Cycloalkyl, Alkenyl, Alkinyl, Aryl steht, die gegebenenfalls substituiert sind,

$R^2$ für Alkyl, Alkenyl, Alkinyl steht, die gegebenenfalls substituiert sind,
$R^3$ für Alkyl, Alkenyl, Alkinyl steht, die gegebenenfalls substituiert sind, mit der Maßgabe, daß mindestens einer der Reste $R^2$ oder $R^3$ für Propargyl steht,
werden hergestellt, indem man

a) Hydroxylaminderivate der Formel II,

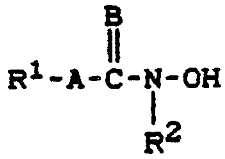

II

in welcher
A, B, $R^1$ und $R^2$ die oben angegebene Bedeutung haben,
mit Verbindungen der Formel III,
$R^3$-X III
in welcher
$R^3$ die oben angegebene Bedeutung hat und
X für Halogen oder p-Tolylsulfonyl steht,
in Gegenwart von Basen umsetzt, oder

b) Thiokohlensäureester der Formel IV,

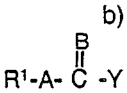

IV

in welcher
A, B, $R^1$ die oben angegebene Bedeutung hat und
Y für Halogen, Azido oder den Rest -O-$R^1$ steht,
mit Hydroxylaminderivaten der Formel V oder ihren Salzen mit Säuren,

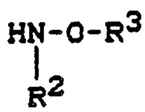

V

in welcher
$R^2$ und $R^3$ die oben angegebene Bedeutung haben,
in Gegenwart von Basen umsetzt, oder

c) für den Fall, daß in Formel I A für S und B für O steht, Hydroxylaminderivate der Formel VI,

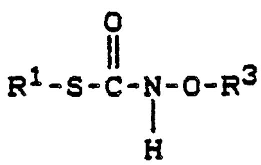

VI

in welcher
$R^1$ und $R^3$ die oben angegebene Bedeutung haben,
mit Verbindungen der Formel VII,
$R^2$-X VII
in welcher
X und $R^2$ die oben angegebene Bedeutung haben,
in Gegenwart von Basen umsetzt, oder

d) Carbamidsäurehalogenide der Formel VIII,

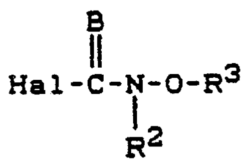

VIII

in welcher
Hal für Halogen steht,
B, $R^2$ und $R^3$ die oben angegebene Bedeutung haben,
mit Verbindungen der Formel IX,
$R^1$-AH IX
in welcher
A, $R^1$ die oben angegebene Bedeutung haben,
in Gegenwart von Basen umsetzt.

e) für den Fall, daß in Formel I A und B für S stehen Dithiocarbamidsäuren bzw. deren Salze der Formel X

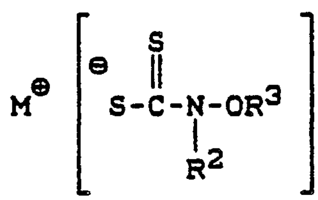

X

in welcher
$R^2$ und $R^3$ die oben angegebene Bedeutung haben
$M^{\oplus}$ für ein Äquivalent eines Kations steht mit Verbindungen der Formel XI
$R^1$ - X XI
in welcher
$R^1$ und X die oben angegebene Bedeutung haben umsetzt.

Die Verbindungen der Formel I eignen sich hervorragend zur Bekämpfung von Endoparasiten, besonders auf dem Gebiet der Veterinärmedizin. Daneben lassen sie sich auch als Insektizide, Fungizide und Herbizide auf den Gebieten der Land- und Fortwirtschaft sowie Haushalt, Hygiene, Vorrats- und Materialschutz einsetzen.

Bevorzugt sind Verbindungen der Formel I,
in welcher
$R^1$ für $C_{1-20}$-Alkyl, $C_{2-20}$-Alkenyl, $C_{3-20}$-Cycloalkyl, $C_{2-20}$-Alkinyl, Phenyl oder Naphthyl steht wobei diese Reste durch einen oder mehrere der folgenden Substituenten substiutiert sein können: Alkyl mit vorzugsweise 1 bis 4, insbesondere 1 oder 2 Kohlenstoffatomen, wie Methyl, Ethyl, n.- und i.-Propyl und n.-, i.-, s.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, s.- und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i-, s.-und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlen stoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; im Falle von Phenyl, für Alkylendioxy mit vorzugsweise 1 oder 2 Kohlenstoffatomen wie Methylendioxy oder Ethylendioxy; im Falle von Phenyl für halogensubstituiertes Alkylendioxy mit vorzugsweise 1 oder 2 Kohlenstoffatomen und vorzugsweise 1 bis 4, insbesondere 2 bis 3 Halogenatomen, wobei

die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy. Weitere Substituenten sind Halogen, vorzugsweise Fluor, Chlor, Brom und Jod, insbesondere Chlor und Brom; Cyano; Nitro; Dialkylamino mit vorzugsweise 1 bis 4, insbesondere 1 oder 2 Kohlenstoffatomen je Alkylgruppe, wie Dimethylamino, Diethylamino, Methyl-n.-butylamino; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder substituiert sein können.

$R^2$ für $C_{1-6}$-Alkyl, $C_{2-6}$-Alkenyl, $C_{2-6}$-Alkinyl steht, die gegebenenfalls durch Halogen, insbesondere Chlor, Fluor, Brom, substituiert sein können,

$R^3$ für $C_{1-6}$-Alkyl, $C_{2-6}$-Alkenyl, $C_{2-6}$-Alkinyl steht, die gegebenenfalls durch Halogen, insbesondere Chlor, Fluor, Brom oder gegebenenfalls substituiertes Aryl substituiert sein können. Als gegebenenfalls substituierte Arylreste kommen insbesondere die bei der Definition des Substituenten $R^1$ genannten Phenyl oder Naphthylreste in Frage, die durch die dort genannten Substituenten substituiert sein können.

Besonders bevorzugt sind Verbindungen der Formel I,

in welcher

$R^1$ für $C_{1-4}$-Alkyl steht, das gegebenenfalls substituiert ist durch Halogen, insbesondere Chlor oder Fluor, $C_{1-4}$-Alkoxy wie Methoxy oder Ethoxy, $C_{1-4}$-Halogenalkoxy wie Trifluormethoxy, $C_{1-4}$-Halogenalkylthio wie Trifluormethylthio, Phenyl, das gegebenenfalls substituiert ist, ferner für Phenyl steht, das gegebenenfalls substituiert ist durch $C_1$-$C_4$-Alkyl, insbesondere Methyl, $C_1$-$C_4$-Alkoxy, insbesondere Methoxy, Ethoxy, $C_1$-$C_4$-Halogenalkoxy, insbesondere Trifluormethoxy, Fluorchlorethoxy, $C_1$-$C_4$-Halogenalkylthio, insbesondere Trifluormethylthio, Fluorchlormethylthio, $C_1$-$C_4$-Alkylthio, insbesondere Methylthio, Halogensulfonyl, insbesondere Fluorsulfonyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Methylendioxy oder Ethylendioxy, die gegebenenfalls durch Fluor oder Chlor substituiert sind, Halogen, insbesondere Fluor oder Chlor, $NO_2$, Phenoxy, das gegebenenfalls durch einen der oben angegebenen Reste substituiert ist,

$R^2$ für $C_{1-6}$-Alkyl, insbesondere Methyl, Ethyl, n-Propyl, i-Propyl, t-Butyl, $C_{2-4}$-Alkenyl, insbesondere Allyl, Chlorpropenyl, Dichlorpropenyl, Butenyl, $C_{2-4}$-Alkinyl, insbesondere Propargyl steht,

$R^3$ für $C_{1-6}$-Alkyl, insbesondere Methyl, Ethyl, n-Propyl, i-Propyl, t-Butyl, $C_{2-4}$-Alkenyl, insbesondere Allyl, Chlorpropenyl, Dichlorpropenyl, Butenyl, $C_{2-4}$-Alkinyl, insbesondere Propargyl steht,

sowie für $C_{1-4}$-Alkyl, das durch gegebenenfalls substituiertes Phenyl substituiert ist, wie insbeson dere gegebenenfalls substituiertes Benzyl, wobei als Substituenten die bei $R^1$ genannten Substituenten des Phenylrestes in Frage kommen.

Ganz besonders bevorzugt sind Verbindungen der Formel I,

in welcher

$R^1$ für $C_{1-4}$-Alkyl, insbesondere Methyl oder Ethyl, Benzyl, Phenyl, das gegebenenfalls durch Halogen, insbesondere Fluor, Chlor, $NO_2$, $CF_3$, $OCF_3$, $SCF_3$, $SCF_2Cl$, $OCH_3$, $OCF_2CF_2H$, $-OCF_2CHFO-$, $-O-CH_2-O$, $-O-CF_2-O$-substituiert ist, steht,

$R^2$ für Methyl, Ethyl, n-Propyl, i-Propyl, Butyl, Pentyl, Allyl, 3-Chlor-prop-2-enyl, 2-Chlor-prop-2-enyl, 3,3-Dichlorprop-2-enyl, But-2-enyl, Propargyl steht,

$R^3$ für Methyl, Ethyl, n-Propyl, i-Propyl, Butyl,Pentyl, Allyl, 3-Chlor-prop-2-enyl, 2-Chlor-prop-2-enyl, 3,3-Dichlorprop-2-enyl, But-2-enyl, Propargyl steht,

sowie für Benzyl steht, das gegebenenfalls durch $C_{1-4}$-Alkyl wie Methyl, Ethyl, Halogen wie Chlor, Fluor, Nitro, $C_{1-2}$-Halogenalkyl wie Trifluoralkyl, $C_{1-2}$-Halogenalkoxy wie Trifluormethoxy, $C_{1-2}$-Halogenalkylthio wie Trifluormethylthio substituiert ist.

Im einzelnen seien folgende Verbindungen der Formel I genannt:

| $R^1$ | $R^3$ | $R^2$ | A | B |
|---|---|---|---|---|
| $C_2H_5$ | $CH_3$- | $-CH_2-CH=CH_2$ | S | O |
| $C_2H_5$ | $C_2H_5$- | $-CH_2-CH=CH_2$ | S | O |
| $C_2H_5$ | $C_3H_7$- | $-CH_2-CH=CH_2$ | S | O |
| $C_2H_5$ | $iC_3H_7$- | $-CH_2-CH=CH_2$ | S | O |
| $C_2H_5$ | $-CH_2-CH=CH_2$ | $-CH_2-CH=CH_2$ | S | O |
| $C_2H_5$ | $-CH_2-CH=CHCl$ | $-CH_2-CH=CH_2$ | S | O |
| $C_2H_5$ | $-CH_2-C=CH$ | $-CH_2-CH=CH_2$ | S | O |
| $C_2H_5$ | $-CH_2-CH=CH-CH_3$ | $-CH_2-CH=CH_2$ | S | O |
| $C_2H_5$ | $-CH_2-CH=CH_2$ | $-CH_2-CH=CH_2$ | O | S |
| $CH_3$- | $CH_3$- | $-CH_2-CH=CH_2$ | O | S |
| $CH_3$- | $C_2H_5$ | $-CH_2-CH-CH_2$ | S | O |
| $CH_3$- | $iC_3H_7$- | $-CH_2-CH=CH_2$ | S | O |
| $CH_3$- | $-CH_2-CH=CH_2$ | $-CH_2-CH=CH_2$ | O | S |
| $CH_3$- | $-CH_2-C\equiv CH$ | $-CH_2-CH=CH_2$ | S | O |

| $R^1$ | $R^3$ | $R^2$ | A | B |
|---|---|---|---|---|
| 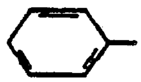 | $CH_3-$ | $-CH_2-CH=CH_2$ | S | O |
| 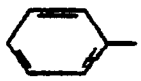 | $C_2H_5-$ | $-CH_2-CH=CH_2$ | S | O |
| 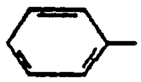 | $iC_3H_7-$ | $-CH_2-CH=CH_2$ | S | O |
| 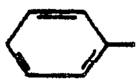 | $nC_3H_7-$ | $-CH_2-CH=CH_2$ | S | O |
| 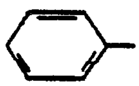 | $-CH_2-CH=CH_2$ | $-CH_2-CH=CH_2$ | S | O |
| 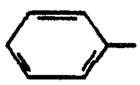 | $-CH_2-CH=CHCl$ | $-CH_2-CH=CH_2$ | S | O |
| 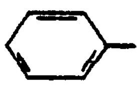 | $-CH_2-C\equiv CH$ | $-CH_2-CH=CH_2$ | S | O |
| 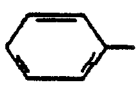 | $-CH_2-CH=CH-CH_3$ | $-CH_2-CH=CH_2$ | S | O |
| $C_2H_5-$ | $CH_3-$ | $-CH_2-C\equiv CH$ | S | O |
| $C_2H_5-$ | $iC_3H_7-$ | $-CH_2-C\equiv CH$ | S | O |
| $C_2H_5-$ | $-CH_2-CH=CH_2$ | $-CH_2-C\equiv CH$ | S | O |
| $C_2H_5-$ | $-CH_2-C\equiv CH$ | $-CH_2-C\equiv CH$ | S | O |
| $C_2H_5-$ | $-CH_2-CH=CHCl$ | $-CH_2-C\equiv CH$ | S | O |
| $C_2H_5-$ | $C_2H_5-$ | $-CH_2-C\equiv CH$ | S | O |
| $C_2H_5-$ | $C_2H_5-$ | $-CH_2-C\equiv CH$ | O | S |

| $R^1$ | $R^3$ | $R^2$ | A | B |
|---|---|---|---|---|
| $C_2H_5$- | $-CH_2-CH=CCl_2$ | $-CH_2-C\equiv CH$ | S | O |
| $C_2H_5$- | $-CH_2-CBr=CHBr$ | $-CH_2-C\equiv CH$ | S | O |
| $C_2H_5$- | $C_2H_5$- | $-CH_2-C\equiv CH$ | S | S |
| $CH_3$- | $C_2H_5$- | $-CH_2-C\equiv CH$ | S | O |
| $CH_3$- | $-CH_2-CH=CH_2$ | $-CH_2-C\equiv CH$ | S | O |
| $CH_3$- | $-CH_2-CH=CHCl$ | $-CH_2-C\equiv CH$ | S | O |
| $CH_3$- | $-CH_2-C\equiv CH$ | $-CH_2-C\equiv CH$ | S | O |
| $CH_3$- | $-CH_2-CH=CH-CH_3$ | $-CH_2-C\equiv CH$ | S | O |
| 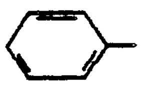 | $CH_3$- | $-CH_2-C\equiv CH$ | O | S |
| 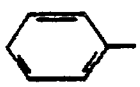 | $C_2H_5$- | $-CH_2-C\equiv CH$ | O | S |
| 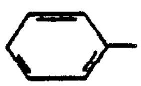 | $-CH_2-CH=CH_2$ | $-CH_2-C\equiv CH$ | S | O |
| 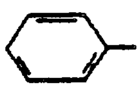 | $-CH_2-C\equiv CH$ | $-CH_2-C\equiv CH$ | S | O |
| 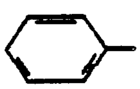 | $-CH_2-CH=CHCl$ | $-CH_2-C\equiv CH$ | S | O |

| $R^1$ | $R^3$ | $R^2$ | A | B |
|---|---|---|---|---|
| 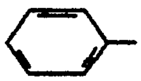 | $CH_3$- | $-CH_2-C{\equiv}CH$ | S | O |
| 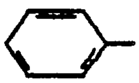 | $C_2H_5$- | $-CH_2-C{\equiv}CH$ | S | O |
| 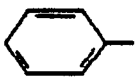 | $C_2H_5$ | $-CH_2-C{\equiv}CH$ | S | S |
| 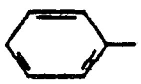 | $C_2H_5$- | $-CH_2-CH{=}CH_2$ | O | S |
| 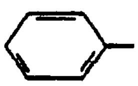 | $C_2H_5$- | $-CH_2-CH{=}CH_2$ | S | S |
| 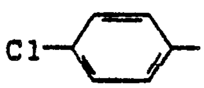 | $-CH_3$- | $-CH_2-C{\equiv}CH$ | S | O |
| 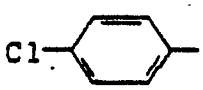 | $-C_2H_5$- | $-CH_2-C{\equiv}CH$ | S | O |
| 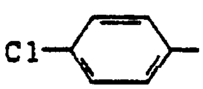 | $-C_2H_5$ | $-CH_2-C{\equiv}CH$ | O | S |
| 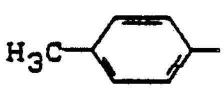 | $C_2H_5$- | $-CH_2-C{\equiv}CH$ | O | S |
| 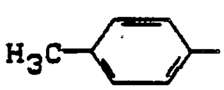 | $C_2H_5$- | $-CH_2-C{\equiv}CH$ | S | O |
| 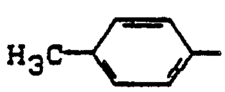 | $C_2H_5$- | $-CH_2-C{\equiv}CH$ | S | S |

Setzt man bei Verfahren 3a als Hydroxylaminderivat der Formel II Benzylthiocarbonyl-N-propargylhydroxylamin und als Verbindung der Formel III Allylbromid ein, so läßt sich der Reaktionsverlauf durch folgendes Formelschema wiedergeben:

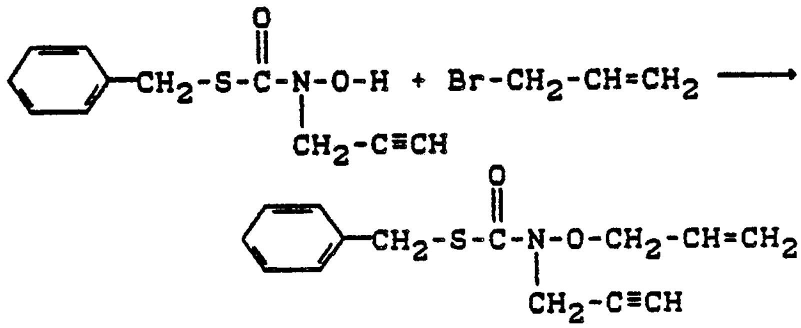

Bevorzugt werden Verbindungen der Formeln II und III eingesetzt, in denen $R^1$, $R^2$ und $R^3$ die bei den Verbindungen der Formel I angegebenen bevorzugten und besonders bevorzugten Bedeutungen haben und X für Chlor, Brom oder p-Tolylsulfonyl steht.

Im einzelnen seien folgende Verbindungen der Formel II genannt:
N-Hydroxy-N-methyl-carbamidsäure-thioethylester
N-Hydroxy-N-methyl-carbamidsäure-thiomethylester
N-Hydroxy-N-allyl-carbamidsäure-thioethylester
N-Hydroxy-N-propargyl-carbamidsäure-thiomethylester
N-hydroxy-N-ethyl-carbamidsäure-thiophenylester
N-Hydroxy-N-3-chlorallyl-carbamidsäure-thioethylester
N-Hydroxy-N-crotyl-carbamidsäure-thioisopropylester
N-Hydroxy-N-propargyl-carbamidsäure-thiophenylester
N-Hydroxy-N-allyl-carbamidsäure-p-thiochlorphenylester
N-Hydroxy-N-propargyl-carbamidsäure-thiocyclopropylester

Die Verbindungen der Formel II sind bekannt oder lassen sich analog zu bekannten Verfahren herstellen (Houben-Weyl, Bd. E4, S. 255, Stuttgart 1983). So kann man z.B. Benzylthiocarbonyl-N-propargyl-hydroxylamin herstellen indem man Benzylthiochlorformiat mit Salzen von N-Propargylhydroxylamin in Gegenwart von Basen umsetzt.

Im einzelnen seien folgende Verbindungen der Formel III genannt: Methyl-, Ethyl-, Propyl-, i-Propyl-, n-Butyl-, s-Butyl-, Allyl-, Crotyl-, 3-Chlorallyl-, 3,3-Dichlorallyl-, 2,3-Dibromallyl-, Propargyl-, 2-Butinylchlorid, -bromid oder -tosylat.

Die Verbindungen der Formel III sind bekannt oder lassen sich analog zu bekannten Verfahren herstellen.

Die Umsetzung erfolgt bei Temperaturen von 0 - 200 °C, bevorzugt bei 20 - 100 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Die Verbindungen der Formeln II und III werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 3b als Thiokohlensäureester der Formel IV Thionochlorameisensäureethylester

und als Hydroxylaminderivat der Formel V N-Allyl-O-propargyl-hydroxylamin ein, so läßt sich der Reaktionsverlauf durch folgendes Formelschema wiedergeben:

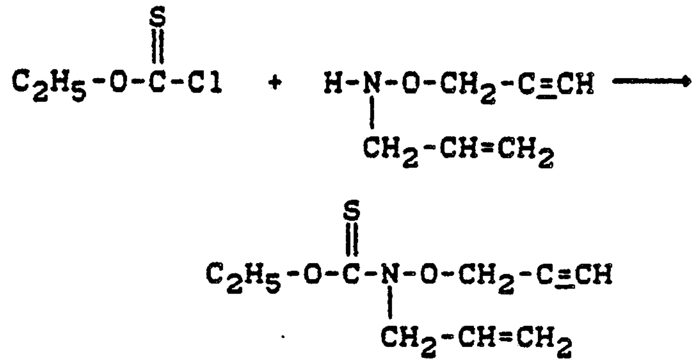

Bevorzugt werden Verbindungen der Formeln IV und V eingesetzt, in denen $R^1$, $R^2$ und $R^3$ die bei den Verbindungen der Formel I angegebenen bevorzugten und besonders bevorzugten Bedeutungen haben und Y für Chlor steht. Die Verbindungen der Formel IV und V sind bekannt oder lassen sich analog zu bekannten Verfahren herstellen (EP-OS 29 171).

Im einzelnen seien folgende Verbindungen der Formel IV genannt: Thionochlorameisensäuremethylester, Chlorameisensäurethioethylester, Thionochlorameisensäure-n-propylester, Dithiochlorameisensäureisopropylester, Thionochlorameisensäure-cyclopropylester, Dithiochlorameisensäurebenzylester, Chlorameisensäure-thiophenylester, Dithiochlorameisensäure-p-chlorphenylester, Chlorameisensäure-thio-p-nitrophenylester, Thionochlorameisensäure-p-tolylester.

Im einzelnen seien folgende Verbindungen der Formel V genannt:
O,N-Diallylhydroxylamin, O,N-Dipropargylhydroxlamin, O-Ethyl-N-allylhydroxlamin, O-Methyl-N-propargylhydroxylamin, O-Propargyl-N-ethylhydroxlamin, O-Allyl-N-methylhydroxylamin, O-Allyl-N-propargylhydroxylamin, O-Crotyl- N-i-propylhydroxylamin, O-3-Chlorallyl-N-propargylhydroxylamin, O-3,3-Dichlorallyl-N-allylhydroxylamin, O-Benzyl-N-3-chlorallylhydroxylamin, O-Methyl-N-2-butinylhydroxylamin.

Die Hydroxylamine der Formel V können in Form ihrer Salze mit Säuren z.B. als Hydrochloride eingesetzt werden.

Die Umsetzung erfolgt bei Temperaturen von -50 - 50 °C, bevorzugt bei 0 °C - 30 °C, besonders bevorzugt bei Raumtemperatur.

Als Verdünnungsmittel kommen Wasser sowie alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Die Verbindungen der Formeln IV und V werden in etwa äquimolarem Verhältnis zueinander eingesetzt. Ein Überschuß der einen oder anderen Komponente bringt keinen wesentlichen Vorteil.

Bevorzugt erfolgt die Umsetzung in wäßriger Lösung der Hydrochloride der Hydroxylaminderivate der Formel V mit Kohlensäureestern der Formel IV in Gegenwart wäßriger anorganischer Basen wie Kalium- oder Natriumhydroxid oder -carbonat. Nach erfolgter Umsetzung wird die Verbindung I in an sich bekannter Weise isoliert, z.B. durch Extraktion mit einem geeigneten Lösungsmittel. Der Extrakt wird mehrfach mit Wasser gewaschen, z.B. über Natriumsulfat getrocknet und dann vom Lösungsmittel befreit. Die Verbindungen der Formel I können anschließend in üblicher Weise, z.B. durch Destillation gereinigt werden.

Setzt man bei Verfahren 3c als Hydroxylaminderivat der Formel VI N-Propargyloxy-carbamidsäurethiobenzylester und als Verbindung der Formel VIII n-Hexylbromid ein, so läßt sich der Reaktionsverlauf durch

folgendes Formelschema wiedergeben:

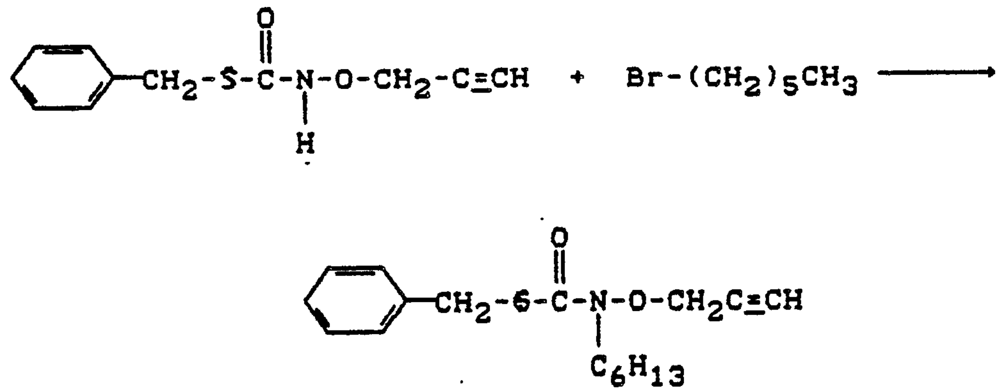

Bevorzugt werden Verbindungen der Formel VI und VII eingesetzt, in denen $R^1$, $R^2$ und $R^3$ die bei den Verbindungen der Formel I angegebenen bevorzugten und besonders bevorzugten Bedeutungen haben und X für Chlor, Brom oder p-Tolylsulfonyl steht. Die Verbindungen der Formeln VI und VII sind bekannt oder lassen sich analog zu bekannten Verfahren herstellen (Houben-Weyl, Bd. E4, S. 258, Stuttgart, 1983). Z.B. läßt sich N-Propargyloxycarbamidsäurethiobenzylester herstellen indem man Benzylthiohydroxyurethan mit Propargylbromid umsetzt.

Im einzelnen seien folgende Verbindungen der Formel VI genannt: N-Ethoxy-carbamidsäure-thioethylester, N-Allyloxycarbamidsäure-thioethylester, N-Propargyloxy-carbamidsäure-thioethylester, N-Crotyloxy-carbamidsäurethiomethylester, N-(3-Chlorallyloxy)-carbamidsäurethiomethylester, N-Propargyloxycarbamidsäure-thiophenylester, N-Allyloxy-carbamidsäure-thioperchlorphenylester, N-2-Butinyloxy-carbamidsäure-thiophenylester.

Im einzelnen seien folgende Verbindungen der Formel VII genannt:
Methyl-, Ethyl-, n-Propyl-, i-Propyl-, n-Butyl-, i-Butyl-, n-Hexyl-, Allyl-, Crotyl-, 3-Chlorallyl-, 3,3- Dichlorallyl-, 2,3-Dibromallyl-, Propargyl-, 2-Butinylchlorid, -bromid oder -tosylat.

Für den Fall, daß Verbindungen der Formel I hergestellt werden sollen, in denen $R^2$ für Methyl oder Ethyl steht, können als Verbindungen der Formel VII auch Dimethylsulfat oder Diethylsulfat eingesetzt werden.

Die Umsetzung erfolgt bei Temperaturen von 0 - 200° C, bevorzugt bei 20 - 130° C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkali hydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-un-decan(DBU), 1,4-Diazabicyclo(2,2,2)octan (DABCO), Diazabicyclo(3,2,0)nonen (DBN).

Die Verbindungen der Formeln VI und VII werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel, z.B. Ether, extrahiert werden. Die Verbindungen der Formel I können anschließend in üblicher Weise, z.B. durch Destillation, gereinigt werden.

Setzt man bei Verfahren 3d als Carbamidsäurehalogenide der Formel VIII N-(2-Butenoxy)-N-propargylcarbamidsäurechlorid und als Verbindung der Formel IX Thiophenol ein, so läßt sich der Reaktionsmechanismus durch folgendes Formelschema wiedergeben:

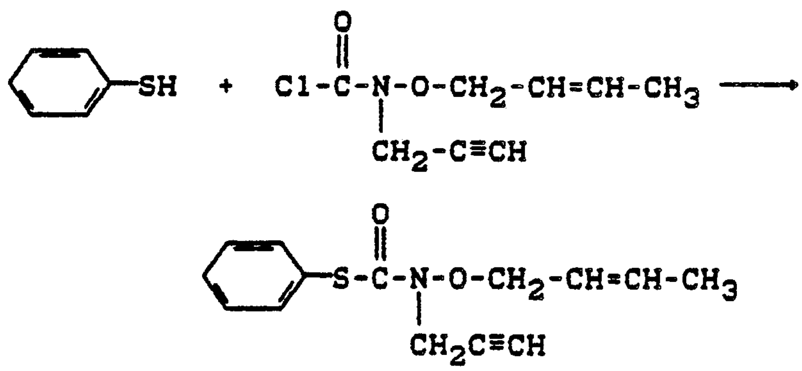

Bevorzugt werden Verbindungen der Formel VIII und IX eingesetzt, in denen $R^1$, $R^2$ und $R^3$ die bei den Verbindungen der Formel I angegebenen bevorzugten und besonders bevorzugten Bedeutungen haben und Hal für Chlor oder Brom steht.

Die Verbindungen der Formeln VIII und IX sind bekannt oder lassen sich analog zu bekannten Verfahren herstellen. (Houben-Weyl, Bd. E4, Stuttgart 1983, S. 258). Z.B. läßt sich N-(2-Butenoxy)-N-propargylcarbamidsäurechlorid herstellen indem man O-Crotyl-N-propargyl-hydroxylamin mit Phosgen umsetzt.

Im einzelnen seien folgende Verbindungen der Formel VIII genannt: N-Methoxy-N-allyl-carbamoylchlorid, N-Allyloxyallylthiocarbamoylchlorid, N-Ethoxy-N-propargyl-carbamoylchlorid, N-Allyloxy-N-propargylthiocarbamoylchlorid, N-Propargyloxy-N-ethyl-thiocarbamoylchlorid, N-Benzyloxy-N-propargyl-carbamoylchlorid, N-Propargyloxy-N-propargyl-carbamoylchlorid.

Im einzelnen seien folgende Verbindungen der Formel IX genannt: Methanol, Ethanol, Propanol, Isopropanol, n-, i-, s- und t.-Butanol, Pentanole, Hexanol, Cyclohexanol, Allylalkohol, Propargylalkhol, Phenol, p-Kresol, p-Chlorphenol, α-Naphthol, p-Nitrochlorphenol, Methyl-, Ethyl-, Propyl-, Isopropyl-, Alkylmercaptan, Thiophenol, o,m,p-Chlor, o,m,p-Methyl, p-Nitro-, 3,4-Dichlorthiophenol.

Die Umsetzung erfolgt bei Temperaturen von -50 - 50 °C, bevorzugt bei 0° - 30 °C, besonders bevorzugt bei Raumtemperatur.

Als Verdünnungsmittel kommen Wasser sowie alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-un-decan(DBU), 1,4-Diazabicyclo(2,2,2)octan (DABCO), Diazabicyclo(3,2,0)nonen (DBN).

Die Verbindungen der Formeln VIII und IX werden in etwa äquimolarem Verhältnis zueinander eingesetzt. Ein Überschuß der einen oder anderen Komponente bringt keinen wesentlichen Vorteil.

Nach erfolgter Umsetzung wird die Verbindung I in an sich bekannter Weise isoliert, z.B. durch Extraktion mit einem geeigneten Lösungsmittel. Der Extrakt wird mehrfach mit Wasser gewaschen, z.B. über Natriumsulfat getrocknet und dann vom Lösungsmittel befreit. Die Verbindungen der Formel I können anschließend in üblicher Weise, z.B. durch Destillation gereinigt werden.

Setzt man bei Verfahren e als Verbindung der Formel X O-Ethyl-N-propargyl-ammonium-dithiocarbamat und als Verbindung der Formel XI Ethylbromid ein, so läßt sich das Verfahren durch folgendes Formelschema darstellen:

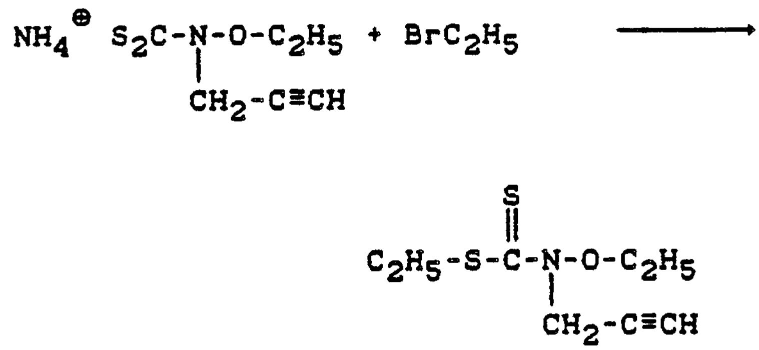

Verbindungen der Formel X sind bekannt bzw. lassen sich analog zu bekannten Verfahren herstellen. Z.B. kann man O-Ethyl-N-propargyl-ammonium-dithiocarbamat erhalten indem man O-Ethyl-N-propargylhydroxylamin in Gegenwart einer Base mit Schwefelkohlenstoff kondensiert.

Bevorzugt werden Verbindungen der Formeln X und XI eingesetzt in denen $R^1$, $R^2$ und $R^3$ die bei den Verbindungen der Formel I angegebenen bevorzugten und besonders bevorzugten Bedeutungen haben und M für Alkali wie Natrium, Kalium, sowie Ammonium, Trimethyl- und Triethylammonium steht. Im einzelnen seien Verbindungen der Formel X genannt:
N-Methoxy-N-proargyl-kaliumdithiocarbamat,
N-Ethoxy-N-propargyl-natriumdithiocarbamat,
N-Allyloxy-N-allyl-triethylammoniumdithiocarbamat,
N-Benzyloxy-N-propargyl-ammoniumdithiocarbamat,
N-Propoxy-N-allyl-natriumdithiocarbamat.

Im einzelnen seien folgende Verbindungen der Formel XI genannt: Methyl-, Ethyl-, Propyl-, i-Propyl-, n-Butyl-, s-Butyl-, Allyl-, Crotyl-, 3-Chlorallyl-, 3,3-Dichlorallyl-, 2,3-Dibromallyl-, Propargyl-, 2-Butinylchlorid, -bromid oder -tosylat.

Die Verbindungen der Formel XI sind bekannt oder lassen sich analog zu bekannten Verfahren herstellen.

Die Umsetzung erfolgt bei Temperaturen von 0 - 200 °C, bevorzugt bei 20 - 100 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-un-decan(DBU), 1,4-Diazabicyclo(2,2,2)octan (DABCO), Diazabicyclo(3,2,0)nonen (DBN).

Die Verbindungen der Formeln X und XI werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Nach erfolgter Umsetzung wird die Verbindung I in an sich bekannter Weise isoliert, z.B. durch Extraktion mit einem geeigneten Lösungsmittel. Der Extrakt wird mehrfach mit Wasser gewaschen, z.B. über Natriumsulfat getrocknet und dann vom Lösungsmittel befreit. Die Verbindungen der Formel I können anschließend in üblicher Weise, z.B. durch Destillation gereinigt werden.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs-

und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:
Aus der Ordnung der Pseudophyllidea z.B.: Diphyllobothrium spp., Spirometra spp., Schistocephalus spp., Ligula spp., Bothridium spp., Diphlogonoporus spp..
Aus der Ordnung der Cyclophyllidea z.B.: Mesocestoides spp., Anoplocephala spp., Paranoplocephala spp., Moniezia spp., Thysanosomsa spp., Thysaniezia spp., Avitel lina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..
Aus der Unterklasse der Monogenea z.B.: Gyrodactylus spp., Dactylogyrus spp., Polystoma spp..
Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp..
Aus der Ordnung der Enoplida z.B.: Trichuris spp., Capillaria spp., Trichomosoides spp., Trichinella spp..
Aus der Ordnung der Rhabditia z.B.: Micronema spp., Strongyloides spp..
Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..
Aus der Ordnung der Oxyurida z.B.: Oxyuris spp., Enterobius spp., Passalurus spp., Syphacia spp., Aspiculuris spp., Heterakis spp..
Aus der Ordnung der Ascaridia z.B.: Ascaris spp., Toxascaris spp., Toxocara spp., Parascaris spp., Anisakis spp., Ascaridia spp..
Aus der Ordnung der Spirurida z.B.: Gnathostoma spp., Physaloptera spp., Thelazia spp., Gongylonema spp., Habronema spp., Parabronema spp., Draschia spp., Dracunculus spp..
Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..
Aus der Ordnung der Gigantorhynchida z.B.: Filicollis spp., Moniliformis spp., Macracanthorhynchus spp., Prosthenorchis spp..
Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.
Zu Labor- und Versuchstieren gehören Mäuse, Ratten, Meerschweinchen, Goldhamster, Hunde und Katzen.
Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln;
Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzen trate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff die Haut durchdringt und systemisch wirkt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen

Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Beispiel A

In vivo Nematodentest

Nippostronglyos brasiliensis / Ratte

Experimentell mit Nippostrongylus brasiliensis infizierte Ratten werden 6 Tage nach der Infektion an drei aufeinanderfolgenden Tagen oral mittels Schlundsonde behandelt. Die Tiere werden 12 Tage nach der Infektion getötet und die Zahl der Parasiten bestimmt. Es wird die Wirkstoffkonzentration angegeben, bei der 95 % der Parasiten abgetötet wurden (effektive Dosis):

| Wirkstoff Beispiel Nr. | effektive Dosis mg/kg |
|---|---|
| 1 | 250 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksme Dosierungen (Dosis effectiva) sind aus der nachfolgenden Tabelle ersichtlich:

| Wirkstoff Beispiel Nr. | Dosis effectiva in mg/kg |
|---|---|
| 2 | 25 |
| 3 | 25 |
| 4 | 25 |

Beispiel C

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie eine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 2 | 10 |
| 4 | 2,5 |

Beispiel D

In vivo Trematodentest

Fasciloa hepatica / Ratte

Experimentell mit Metacercarien von Fasciola hepatica infizierte Ratten wurden nach der Infektion oral mittels Schlundsonde an drei aufeinanderfolgenden Tagen behandelt. Die Tiere wurden 2 Wochen nach der Infektion getötet und die Zahl der jugendlichen Leberegel im Leberparenchym bestimmt.

In der folgenden Tabelle wird angegeben, welche Dosis (effektive Dosis) erforderlich ist, um 95 % Parasitenreduktion in Vergleich zu einer unbehandelten Kontrolle zu erreichen.

| Wirkstoff Beispiel Nr. | Effektive Dosis $ED_{95}$ (mg/kg x 3) |
|---|---|
| 1 | 100 |
| 2 | 25 |
| 4 | 250 |
| 6 | 50 |
| 7 | 100 |

Herstellungsbeispiele

a) Allgemeine Vorschrift zur Herstellung der N-Organooxycarbamidsäureester der Formel I: gemäß Verfahren 3a)

0,1 Mol des N-Alkyl-N-hydroxi-carbamid-säureesters der Formel II und 0,1 Mol des Alkylierungsmittels der Formel III werden zu einer Lösung von 6 g Kaliumhydroxid in 30 ml Ethanol gegeben und 5h am Rückfluß gekocht. Der Alkohol wird abdestilliert und der rohe Ester mit Ether extrahiert. Nach Abdampfen des Ethers wird der Rückstand im Vakuum destilliert.

b) Allgemeine Vorschrift gemäß Verfahren 3b)

0,1 Mol des N,O-Dialkylhydroxylaminhydrochlorid der Formel (V) und 25 ml Wasser und 100 ml Ether werden vorgelegt und bei Raumtemperatur unter kräftigem Rühren mit 0,2 Mol Natriumhydrogencarbonat versetzt. Man rührt 40 min. bei Raumtemperatur nach, kühlt dann auf 0 °C und tropft 0,1 Mol des Chlorameisensäurethioesters der Formel IV zu. Anschließend wird 24h bei Raumtemperatur nachgerührt, die Etherphase abgetrennt, über $Na_2SO_4$ getrocknet und eingeengt. Der Rückstand wird analysiert (Gaschromatographie) und gegebenenfalls im Vakuum destiliert.

c) Allgemeine Vorschrift gemäß Verfahren 3c

0,1 Mol des N-Alkoxythiocarbamidsäureesters der Formel VI und 0,1 Mol Natronlauge werden in 100 ml Methanol und 10 ml Wasser vorgelegt und bei Raumtemperatur mit 0,1 Mol des Alkylierungsreagenzes der Formel VII versetzt. Man rührt 48h bei Raumtemperatur nach, dampft den Alkohol ab und extrahiert mit Ether. Die Etherextrakte werden getrocknet, der Ether abdestilliert und der Rückstand destilliert.

Analog einem der Verfahren 3a-c werden die Wirkstoffe der folgenden Beispiele erhalten:

**Beispiele**

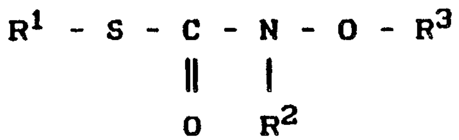

| Bsp. Nr. | $R^1$ | $R^3$ | $R^2$ | NMR - Daten |
|---|---|---|---|---|
| 1 | $C_2H_5$ | $C_2H_5$- | $CH_2$=CH-$CH_2$- | δ = 1,3 ppm (2t,6H), 5,2 ppm (m,2H), 2,85 ppm (q,2H) 5,85 ppm (m,1H) |
| 2 | $C_2H_5$ | $C_2H_5$- | HC≡C-$CH_2$- | δ = 1,3 ppm (2t,6H), 2,3 ppm (t,1H), 2,85 ppm (q,2H) |
| 3 | $C_2H_5$ | $CH_2$=CH-$CH_2$- | $CH_2$=CH-$CH_2$- | δ = 2,8 ppm (q,2H), δ = 5,3 ppm (m,4H) δ = 5,9 ppm (m,2H) |
| 4 | $C_2H_5$- | $CH_2$=CH-$CH_2$ | CH≡C-$CH_2$ | δ = 2,25 ppm (t,1H), δ = 5,4 ppm (m,2H) δ = 6,0 ppm (m,1H) |
| 5 | $CH_3$- | $CH_2$=CH-$CH_2$- | $CH_2$=CH-$CH_2$- | δ = 2,3 ppm (S,3H), δ = 5,3 ppm, (m,2H) δ = 5,9 ppm (m,2H) |

**Beispiele** - Fortsetzung

| Bsp. Nr. | $R^1$ | $R^3$ | $R^2$ | NMR - Daten |
|---|---|---|---|---|
| 6 | $CH_3-$ | $CH_2=CH-CH_2-$ | $HC\equiv C-CH_2-$ | δ = 2,3 ppm (s,3H + t,1H), δ = 5,4 ppm (m,2H), δ = 6,05 ppm (m,1H) |
| 7 | $nC_4H_9-$ | $CH_2 = CH-CH_2-$ | $HC\equiv C-CH_2-$ | δ = 1,3 ppm (t,1H), δ = 5,4 ppm (m,2H) δ = 6,0 ppm (m,1H) |
| 8 | $nC_4H_9-$ | $CH_2=CH-CH_2-$ | $ClHC=CH-CH_2-$ | δ = 5,35 ppm (m,2H), δ = 6 ppm (m,2H) δ = 6,25 ppm (m,1H) |
| 9 | $C_2H_5-$ | $CH_2=CH-CH_2-$ | $H_2C=CH-CH_2-$ | δ = 2,9 ppm (q,2H), δ = 4,35 ppm (s,2H), δ = 5,4 ppm (m,4H), δ = 5,95 ppm (m,1H) |
| 10 | $C_2H_5-$ | $CH_2=CH-CH_2-$ | $ClCH=CH-CH_2-$ | δ = 2,85 ppm (q,2H), δ=4,1 ppm (d,2H), δ = 5,35 ppm (m,2H), δ = 5,35 ppm (m,2H), δ = 6,25 ppm (m,1H) |

**Beispiele** - Fortsetzung

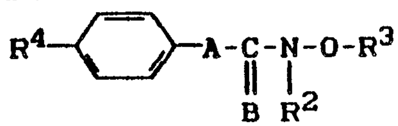

| Bsp. Nr. | A | B | $R^2$ | $R^3$ | $R^4$ | NMR - Daten |
|---|---|---|---|---|---|---|
| 11 | O | S | $HC\equiv C-CH_2-$ | $C_2H_5-$ | H | δ= 2,4 ppm (t,1H), δ = 4,3 ppm (9,2H)<br>δ = 4,85 ppm (d,2H), δ = 1,35 ppm (t,3H) |
| 12 | S | O | $HC\equiv C-CH_2-$ | $C_2H_5$ | Cl- | δ = 1,4 ppm (t,3H), δ = 2,3 ppm (t,1H)<br>δ = 4,25 ppm (q,2H), δ = 4,35 ppm (S/2H) |
| 13 | S | O | $HC\equiv C-CH_2-$ | $CH_3-$ | Cl- | δ = 2,3 ppm (1H/t), δ = 3,95 ppm (3H/S)<br>δ = 4,3 ppm (D,2H) |
| 14 | O | S | $HC\equiv C-CH_2-$ | $CH_3-$ | H- | δ = 2,35 ppm (1H,t), δ = 4,0 ppm (3H,s)<br>δ = 4,85 ppm (2H,d) |

## Ansprüche

1. Verwendung von schwefelhaltigen N-Organooxycarbamidsäureester der Formel I,

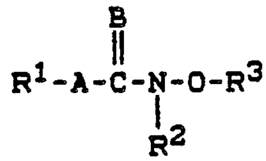

I

in welcher
A und B für O oder S stehen , mit der Maßgabe, daß mindestens einer der Reste A oder B für S stehen muß.
$R^1$ für Alkyl, Cycloalkyl, Alkenyl, Alkinyl, Aryl steht, die gegebenenfalls substituiert sein können,
$R^2$ für Alkyl, Alkenyl, Alkinyl steht, die gegebenenfalls substituiert sein können,
$R^3$ für Alkyl, Alkenyl, Alkinyl steht, die gegebenenfalls substituiert sein können,
zur Bekämpfung von Endoparasiten.

2. Es wurden neue schwefelhaltige N-Organooxycarbamidsäureester der Formel I gefunden,

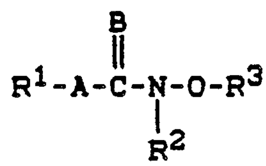

I

A und B für O oder S stehen, mit der Maßgabe, daß mindestens einer der Reste A oder B für S stehen muß.
$R^1$ für Alkyl, Cycloalkyl, Alkenyl, Alkinyl, Aryl steht, die gegebenenfalls substituiert sind,
$R^2$ für Alkenyl, Alkinyl steht, die gegebenenfalls substituiert sind,
$R^3$ für Alkyl, Alkenyl, Alkinyl steht, die gegebenenfalls substituiert sind mit der Maßgabe, daß mindestens einer der Reste $R^2$ oder $R^3$ für Propargyl steht.

3. Verfahren zur Herstellung von schwefelhaltigen N-Organooxycarbamidsäureester der Formel I,

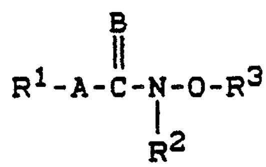

I

in welcher
A und B für O oder S stehen, mit der Maßgabe, daß mindestens einer der Reste A oder B für S stehen muß.
$R^1$ für Alkyl, Cycloalkyl, Alkenyl, Alkinyl, Aryl steht, die gegebenenfalls substituiert sind,
$R^2$ für Alkyl, Alkenyl, Alkinyl steht, die gegebenenfalls substituiert sind,
$R^3$ für Alkyl, Alkenyl, Alkinyl steht, die gegebenenfalls substituiert sind, mit der Maßgabe, daß mindestens einer der Reste $R^2$ oder $R^3$ für Propargyl steht,
werden hergestellt, indem man

a) Hydroxylaminderivate der Formel II

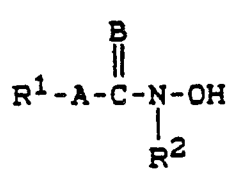

II

in welcher
A, B, $R^1$ und $R^2$ die oben angegebene Bedeutung haben,
mit Verbindungen der Formel III,
$R^3$-X III
in welcher
$R^3$ die oben angegebene Bedeutung hat und
X für Halogen oder p-Tolylsulfonyl steht,
in Gegenwart von Basen umsetzt, oder

b) Thiokohlensäureester der Formel IV,

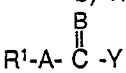 IV

in welcher
A, B, $R^1$ die oben angegebene Bedeutung hat und
Y für Halogen, Azido oder den Rest -O-$R^1$ steht,
mit Hydroxylaminderivaten der Formel V oder ihren Salzen mit Säuren,

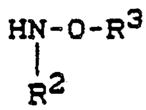 V

in welcher
$R^2$ und $R^3$ die oben angegebene Bedeutung haben,
in Gegenwart von Basen umsetzt, oder für den Fall, daß in Formel I A für S und B für O steht

c) Hydroxylaminderivate der Formel VI,

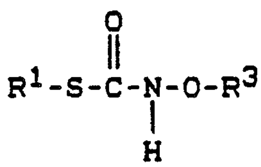 VI

in welcher
$R^1$ und $R^3$ die oben angegebene Bedeutung haben,
mit Verbindungen der Formel VII,
$R^2$-X VII
in welcher
X und $R^2$ die oben angegebene Bedeutung haben,
in Gegenwart von Basen umsetzt, oder

d) Carbamidsäurehalogenide der Formel VIII,

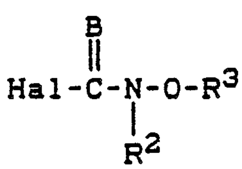 VIII

in welcher
Hal für Halogen steht,
B, $R^2$ und $R^3$ die oben angegebene Bedeutung haben,
mit Verbindungen der Formel IX,
$R^1$-AH IX
in welcher
A, $R^1$ die oben angegebene Bedeutung haben,
in Gegenwart von Basen umsetzt,

e) für den Fall, daß in Formel I A und B für S stehen, Dithiocarbamidsäuren der Formel X

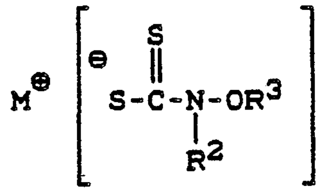

X

in welcher
$R^2$ und $R^3$ die oben angegebene Bedeutung haben
$M^\oplus$ für ein Äquivalent eines Kations steht mit Verbindungen der Formel XI
$R^1$ - X XI
in welcher
$R^1$ und X die oben angegebene Bedeutung haben umsetzt.

4. Endoparasitizide Mittel, gekennzeichnet durch einen Gehalt an mindestens einem schwefelhaltigen N-Organooxycarbamidsäureester der Formel (I) gemäß Anspruch 1.

5. Verfahren zur Herstellung von endoparasitiziden Mitteln, dadurch gekennzeichnet, daß man schwefelhaltige N-Organooxycarbamidsäureester der Formel (I) gemäß Anspruch 1 mit Streckmitteln und/oder oberflächenaktiven Mitteln vermischt.

6. Verwendung von schwefelhaltigen N-Organooxycarbamidsäureestern der Formel (I) gemäß Anspruch 1 zur Herstellung von endoparasitiziden Mitteln.

Europäisches Patentamt

# EUROPÄISCHER RECHERCHENBERICHT

Nummer der Anmeldung

EP 89 10 1610

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS Band 69, August-September 1968, Seite 2484, Zusammenfassung Nr. 26802n, Columbus, Ohio, USA; N.G. ROZHKOVA et al.:"O-alkyl hydroxylamine derivatives of esters of thio-carbonic acid" --- | 2 | C 07 C 155/02<br>C 07 C 155/03<br>C 07 C 155/08<br>C 07 C 155/09<br>A 01 N 47/24 |
| A | EP-A-0 125 205 (CIBA-GEIGY) * Tabelle 1, Verbindung 1.49, Tabelle 2, Verbindungen 2.337,2.338,2.339 * --- | 2 | |
| A | FR-A-2 313 924 (SANKYO) * Anspruch 11 * --- | 1 | |
| A | GB-A-2 191 194 (E.R. SQUIBB & SONS) * Tabelle, Seite 6 * --- | 1 | |
| A | US-A-3 609 175 (S.B. RICHTER) * Zusammenfassung * --- | 1 | |
| A | US-A-3 301 885 (S.B. RICHTER et al.) * Spalte 5, Zeilen 18-37 * ----- | 1 | **RECHERCHIERTE SACHGEBIETE (Int. Cl.4)**<br>C 07 C 155/02<br>C 07 C 155/03<br>C 07 C 155/08<br>C 07 C 155/09<br>A 01 N 47/24 |

Der vorliegende Recherchenbericht wurde für alle Patentansprüche erstellt

| Recherchenort | Abschlußdatum der Recherche | Prüfer |
|---|---|---|
| BERLIN | 03-05-1989 | KAPTEYN H G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)